# EUROPEAN PATENT APPLICATION

(11) **EP 4 449 981 A1**
(43) Date of publication of application: **23.10.2024**
(21) Application number: 23168247.7
(22) Date of filing: 17.04.2023
(51) Int. Cl.: A61B 5/024, A61B 5/1455, A61B 5/00

(54) **WEARABLE SLEEP TRACKER DEVICE WITH ENVIRONMENT AMBIENT LIGHT SENSING**

(71) Applicant: Koninklijke Philips N.V., 5656 AG Eindhoven (NL)
(72) Inventor: VAN DEN ENDE, Daan Anton, 5656AG Eindhoven (NL)
(74) Representative: Philips Intellectual Property & Standards

(57) **Abstract**

A sleep tracker wearable device (10) with ambient light artefact detection and removal when worn by a user comprises an optical sensor/emitter assembly (12), a spacer (14), and a controller (16). The optical sensor/emitter assembly (12) includes an optical sensor (18) with a sensor surface (20) configured for receiving optical signals including ambient light. The spacer (14) is disposed between the sensor surface (20) of the optical sensor/emitter assembly and a skin surface (22) of the user during operation, further for providing a given contact pressure and/or separation distance between the skin and sensor surfaces as a function of an adjustable height of the spacer. The controller (16) controls at least one measurement modality of the sleep tracker wearable device (10). In addition, the controller (16) is coupled to the optical sensor/emitter assembly (i) for detecting background lighting conditions based on the optical signals and (ii) for removing artefacts in the optical signals attributable to an external interaction with or perturbation of a natural progression of ambient lighting level.

## Description

### BACKGROUND

The present embodiments relate generally to wearable sleep tracker devices and more particularly, to a wearable sleep tracker device with environment ambient light sensing and a method thereof.

There are many factors that can affect and impact the quality of a person's sleep. For example, personal factors such as stress, food or sleep deprivation can affect sleep significantly. On the other hand, the surroundings can also have a significant influence on a person's sleep. Several key parameters that determine the right environment for sleep quality include sound, temperature and light levels. A dark environment is paramount for good sleep and levels higher than 1 lux could already be detrimental for sleep. Many bedrooms have poorly closing curtains which do not block sunlight efficiently enough, resulting in too short or poor-quality sleep, especially in summer months.

Many wearable devices currently have a sleep tracking function, which provides an estimation of a user's sleep. Examples include fitness trackers/smartwatches such as Fitbit^{™}, Garmin^{™}, or Apple^{™} watch, or headbands such as a Philips Smartsleep Deep Sleep Headband or a Rhythm Dreem Headband. The value of sleep tracking is shifting from monitoring only to targeted advice based on personal sleep data. The specificity of the advice is also related to meta-data from surroundings or personal data that can be coupled to the sleep data from the sleep tracker using AI (artificial intelligence) algorithms, to enable user specific information with the highest value. Meta-data often requires manual user input or it is provided by other sensors, often from connected devices.

Meta-data often requires manual user input which is a hassle and is intermittent and error-prone when the meta data is provided by other sensors, often from connected devices. However, reliable coupling of these sensors' data is not straightforward, both in terms of technical issues (e.g., accurate time-syncing) as well as practical issues (e.g., due to real-time information sharing, legal agreements, licensing, etc.).

As indicated, ambient light conditions in a bedroom have a big influence on sleep quality and quantity of a person resting in the bedroom, as well as influencing going to sleep and waking up. With respect to ambient lighting conditions, bedside sensors are well suited for ambient light sensing since they are statically positioned with a sensor which has an open view to its surroundings and therefore produces a stable output signal. However, not all people have a bedside sensor which records the ambient lighting level in their bedroom. The Somneo^{™} bedside light has such an ambient lighting level sensing feature.

Sleep wearable devices, on the other hand, although often equipped with optical sensors (i.e., for PPG, SpOz sensing), generally do not actively measure ambient lighting conditions. This is because the wearable device is attached to the user and moves when the user changes body position, giving unnatural artefacts in the light sensor signal (or optical sensor output signal), which disturbs an ambient light sensing quality. Also, the sensor of the sleep wearable device is specifically aimed at measuring reflected (or transmitted) light from the skin and the optical unit is therefore often designed in such a way that the influence of ambient lighting conditions is minimized, i.e., kept as small as possible. Additionally, the sleep wearable, when attached to the user, moves when the user changes body position. This movement gives rise to unnatural artefacts in the light sensor signal, which would disturb an ambient light sensing quality.

One known ambient light sensor, e.g., an OPT3006 available from Texas Instruments, was developed for applications needing to measure light in connection with smart lighting devices or mobile devices. In one application, the ambient light sensor is used in a circuit for adapting display brightness to ambient conditions. In particular, such sensors are dedicated ambient lighting detectors which are used as input to control the brightness of a display. Furthermore, the sensor is situated on the front facing area of the smart lighting device, and/or wearable or mobile device.

Accordingly, an improved method and apparatus for overcoming the problems in the art is desired.

### SUMMARY

In accordance with one aspect, a wearable sleep tracker device with an optical light sensor is disclosed which includes a spacer device configured to allow light to pass through to the optical light sensor in combination with an artefact handling mechanism to correct for user movements and provide reliable environment ambient light sensing/monitoring with the wearable sleep tracker device (e.g., in a bedroom).

According to one embodiment, a sleep tracker wearable device with ambient light artefact detection and removal when worn by a user comprises an optical sensor/emitter assembly, a spacer, and a controller. The optical sensor/emitter assembly includes an optical sensor with a sensor surface configured for receiving optical signals including ambient light. The spacer is disposed between the sensor surface of the optical sensor/emitter assembly and a skin surface of the user during operation. The spacer is configured for providing a given contact pressure and/or separation distance between the skin and sensor surfaces as a function of an adjustable height of the spacer. The controller is configured for controlling at least one measurement modality (e.g., a physiological and/or sleep related measurement modality including at least one modality that utilizes the optical sensor/emitter assembly for emitting and receiving optical signals) of the sleep tracker wearable device. The controller is coupled to the optical sensor/emitter assembly (i) for detecting background lighting conditions based on the optical signals (e.g., the optical signals being representative of background lighting conditions including ambient light) and (ii) for removing artefacts in the optical signals (e.g., wherein the artefacts in the optical signals comprise artefacts attributable to an external interaction with or perturbation of ambient lighting level resulting from movement or repositioning of the sleep tracker wearable device during operation).

In another embodiment, the controller is configured for applying a low pass filter in combination an event detector over a background optical signal of the optical sensor. The event detector is a based on a high pass filter in combination with a threshold value to detect movement spikes in the background optical signal. In one embodiment, the threshold value is calibrated or determined as a global setting related to a floor noise level of the optical sensor. In an absence of movement, no spikes in monitored ambient light levels are detected and a background light level will gradually increase with time as the ambient light levels increase. The controller further determines if the increase in ambient light level corresponds with daylight data (e.g., sunrise timing) as influenced from outside light conditions in contrast to ambient light conditions other than daylight (i.e., during night-time while sleeping).

In yet another embodiment, in a presence of movement, spikes in monitored ambient light levels are detected after which the controller adjusts the detected ambient light levels for continuity (i.e., to match corresponding ambient light levels before and after a respective detected movement spike within a given time interval), so that the ambient light level data remains continuous (i.e., without movement spikes) and can be compared to the daylight data. The controller further determines if the increase in ambient light level corresponds with daylight data (e.g., sunrise timing) as influenced from outside light conditions in contrast to ambient light conditions other than daylight (e.g., during night-time while sleeping).

According to another embodiment, the spacer includes a means for changing a proximity of the sensor surface to the skin surface via the adjustable height. During night-time sleep measurements, the adjustable height is changed from a first height to a second height greater than the first height, wherein a contact pressure between the sensor surface and the skin surface is lower and/or separation distance is higher which results in a slightly higher amount of transmitted ambient light to the optical sensor. During other than night-time sleep measurements, the adjustable height is changed to the first height, wherein the contact pressure between the sensor surface and the skin surface is higher and/or separation distance is lower which results in a slightly lower amount of transmitted ambient light to the optical sensor. In one embodiment, the first height is 1 mm and the second height is greater than 1 mm up to 2 mm.

In another embodiment, the spacer comprises one selected from the group consisting of (i) a spring-loaded spacer geometry, (ii) a manually activated spacer geometry and (iii) a spacer geometry coupled to a manual sleep intent marker. The spring-loaded spacer geometry is responsive to an activation via a controllable switch (e.g., a spacer button press) for releasing the spring-loaded spacer geometry from the first height to the second height. The manually activated spacer geometry is activated via turning a knob (e.g., in one of a clockwise or counter-clockwise direction) for protruding the spacer geometry from the first height to the second height. Lastly, the spacer geometry moves from the first height to the second height in response to a user setting and/or activating the manual sleep intent marker to designate an intent to sleep. One example of a manual sleep intent marker may comprise a user selectable feature of the wearable device in the form of any user activated button or something similar (e.g., a toggle, check mark, or the like) intended to let the wearable device know when the wearer is intending to start sleeping.

In a further embodiment, the means for changing the proximity of the sensor surface to the skin surface via the adjustable height comprises an actuator configured to adjust a thickness of the spacer between a first thickness and a second thickness, larger than the first thickness. The actuator (i) expands the spacer thickness to the second thickness in low ambient lighting conditions and/or a detected sleep condition and (ii) retracts the spacer thickness to the first thickness, in other than low ambient lighting conditions and/or the detected sleep condition. In one embodiment, the actuator is controlled as a function of a background optical signal of the optical sensor. In another embodiment, the actuator comprises an optically active smart material which is responsive to an optical input (e.g., of ambient light level or intensity).

According to yet another embodiment, the sleep tracker wearable device further comprises a position sensor configured to provide information on an angular orientation of the sleep tracker wearable device. In this embodiment, the controller is further configured for correcting the optical signals for ambient light levels based on the angular orientation information. The position sensor may comprise one or more of accelerometer, a gyroscope, an inertial measurement unit (IMU), or any combination thereof. An IMU describes a collection of measurement tools. When installed in the wearable device, these tools can capture data about the device's movement. IMUs contain sensors such as accelerometers, gyroscopes, and magnetomerters. The accelerometer may comprise, for example, a 3-axis or 6-axis accelerometer.

In another embodiment, the controller may further be configured for distinguishing between (i) short-term optical signal changes due to a user movement and (ii) rapid external optical signal changes due to other than the user movement. In addition, the controller is configured for improving a quality of ambient light meta-data derived from the optical sensor signal based on the distinction between the short-term optical signal changes and the rapid external optical signal changes.

According to one embodiment, the controller is further configured for implementing an artificial intelligence algorithm for outputting targeted user-specific sleep advice (e.g., of a highest value) based on (i) sleep data obtained via the at least one measurement modality, (ii) personal data coupled to the sleep data, and (iii) meta-data relating to surroundings of the sleep tracker wearable device based on the detected background lighting conditions.

In another embodiment, a method for ambient light artefact detection and removal in a sleep tracker wearable device comprises: receiving, via an optical sensor/emitter assembly having an optical sensor with a sensor surface, optical signals including ambient light; disposing a spacer between the sensor surface of the optical sensor/emitter assembly and a skin surface of the user during use, wherein the spacer is configured for providing a given contact pressure between the skin and sensor surfaces as a function of an adjustable height of the spacer; and controlling, via a controller coupled to the optical sensor/emitter assembly, at least one measurement modality (e.g., a physiological and/or sleep related measurement modality including at least one modality that utilizes the optical sensor/emitter assembly for emitting and receiving optical signals) of the sleep tracker wearable device. The controlling includes (i) detecting background lighting conditions based on the optical signals (e.g., the optical signals being representative of background lighting conditions including ambient light) and (ii) removing artefacts in the optical signals (e.g., wherein the artefacts in the optical signals comprise artefacts attributable to an external interaction with or perturbation of a natural progression of ambient lighting level resulting from movement or repositioning of the sleep tracker wearable device during operation).

According to another embodiment, the method further comprises wherein disposing the spacer includes changing a proximity of the sensor surface to the skin surface via the adjustable height. During night-time sleep measurements, the adjustable height is changed from a first height to a second height greater than the first height, wherein a contact pressure between the sensor surface and the skin surface is lower and/or separation distance is higher which results in a slightly higher amount of transmitted ambient light to the optical sensor. During other than night-time sleep measurements, the adjustable height is changed to the first height, wherein the contact pressure between the sensor surface and the skin surface is higher and/or separation distance is lower which results in a slightly lower amount of transmitted ambient light to the optical sensor.

In another embodiment, the method further comprises configuring a position sensor to provide information on an angular orientation of the sleep tracker wearable device; and correcting, via the controller, the optical signals for ambient light levels based on the angular orientation information. In yet another embodiment, the method further comprises implementing, via the controller, an artificial intelligence algorithm for outputting targeted user-specific sleep advice (e.g., of a highest value) based on (i) sleep data obtained via the at least one measurement modality (e.g., physiological and/or sleep related measurement modality) of the sleep tracker wearable device, (ii) personal data coupled to the sleep data, and (iii) meta-data relating to surroundings of the sleep tracker wearable device based on the detected background lighting conditions.

Still further advantages and benefits will become apparent to those of ordinary skill in the art upon reading and understanding the following detailed description.

### BRIEF DESCRIPTION OF THE DRAWINGS

The embodiments of the present disclosure may take form in various components and arrangements of components, and in various steps and arrangements of steps. Accordingly, the drawings are for purposes of illustrating the various embodiments and are not to be construed as limiting the embodiments. In the drawing figures, like reference numerals refer to like elements. In addition, it is to be noted that the figures may not be drawn to scale.
Fig. 1 is a block diagram view of a sleep tracker wearable device with ambient light artefact detection and removal according to an embodiment of the present disclosure;
Fig. 2 is a perspective view of a sleep tracker wearable device with ambient light artefact detection and removal according to an embodiment of the present disclosure;
Fig. 3 is a bottom view of a sleep tracker wearable device with ambient light artefact detection and removal according to an embodiment of the present disclosure;
Fig. 4 is a graph view illustration of an output signal of the optical sensor illustrating a difference between a long-term change in the signal and a short abrupt change according to an embodiment of the present disclosure;
Fig. 5 is a graph view illustration of an output signal of the optical sensor as a function of time for a case without movement of the sleep tracker wearable device according to an embodiment of the present disclosure;
Fig. 6 is a graph view illustration of an output signal of the optical sensor as a function of time for a case of movement of the sleep tracker wearable device, and a stitched signal, according to an embodiment of the present disclosure;
Fig. 7 is a graph view illustration of sensor output from an optical sensor of a sleep tracker wearable device under daylight conditions (overcast sky), wherein from 10-30 seconds the wearable device is on the user's wrist facing a window, from 30-60 seconds the wearable device is covered by placing the wearable device under a heavy blanket, and after 60 seconds the blanket is removed and the wearable device is held in an opposite direction (i.e., facing away from the window, according to an embodiment of the present disclosure; and
Fig. 8 is a block diagram view of a sleep tracker wearable device with ambient light artefact detection and removal according to another embodiment of the present disclosure.

### DETAILED DESCRIPTION

The embodiments of the present disclosure and the various features and advantageous details thereof are explained more fully with reference to the non-limiting examples that are described and/or illustrated in the drawings and detailed in the following description. It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be omitted so as to not unnecessarily obscure the embodiments of the present disclosure. The examples used herein are intended merely to facilitate an understanding of ways in which the embodiments of the present may be practiced and to further enable those of skill in the art to practice the same. Accordingly, the examples herein should not be construed as limiting the scope of the embodiments of the present disclosure, which is defined solely by the appended claims and applicable law.

It is understood that the embodiments of the present disclosure are not limited to the particular methodology, protocols, devices, apparatus, materials, applications, etc., described herein, as these may vary. It is also to be understood that the terminology used herein is used for the purpose of describing particular embodiments only, and is not intended to be limiting in scope of the embodiments as claimed. It must be noted that as used herein and in the appended claims, the singular forms "a," "an," and "the" include plural reference unless the context clearly dictates otherwise.

Unless defined otherwise, all technical and scientific terms used herein have the same meanings as commonly understood by one of ordinary skill in the art to which the embodiments of the present disclosure belong. Preferred methods, devices, and materials are described, although any methods and materials similar or equivalent to those described herein can be used in the practice or testing of the embodiments.

Various embodiments of the present disclosure relate to a system and method for using the background output of a photodiode that is part of a photoplethysmography (PPG) or oxygen saturation (SpOz) sensor as an indicator for ambient lighting conditions at night. The system includes a wearable sleep tracker with an optical unit (e.g., optical sensor/emitter assembly) that includes an optical sensor. The background ambient light level measured by the optical sensor is used to determine changes in background ambient light levels. The signal is filtered to separate long-term and short-term effects (e.g., user movement effects) and can be combined with other sensors (e.g., accelerometers) to more accurately determine the difference between ambient light changes and user movement induced changes in the background signal.

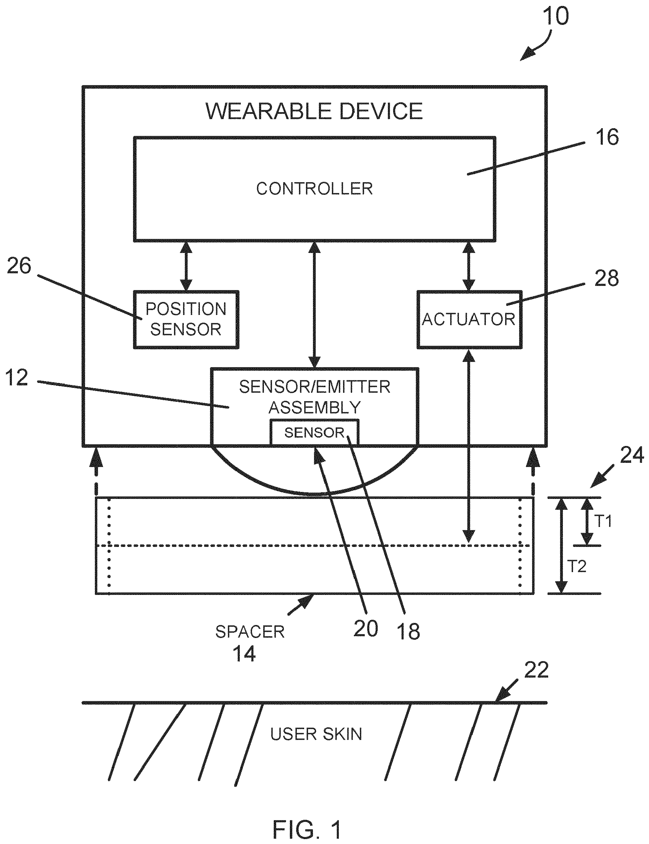

Turning now to Fig. 1, there is shown a block diagram view of a sleep tracker wearable device 10 with ambient light artefact detection and removal according to an embodiment of the present disclosure. The sleep tracker wearable device 10 comprises an optical sensor/emitter assembly 12, a spacer 14, and a controller 16. The optical sensor/emitter assembly 12 includes an optical sensor 18 with a sensor surface 20 configured for receiving optical signals including ambient light. The spacer 14 is coupled to a surface of the wearable device 10, and disposed between the sensor surface 20 of the optical sensor/emitter assembly 12 and a skin surface 22 of the user during operation. The spacer 14 is configured for providing a given contact pressure and/or separation distance between the skin and sensor surfaces as a function of an adjustable height of the spacer 14. The 'pressure' mentioned herein originates from the fact that the sensor is encapsulated in a dome shaped transparent housing, so contact pressure is related to how far this housing is pushed into the skin, which directly influences the light influx in the sensor, based upon experiments. For zero contact pressure between the skin and sensor surface (i.e., at a small separation distance), the method was determined to still work.

With reference still to Fig. 1, the spacer 14 is configured for providing a given contact pressure and/or separation distance between the skin 22 and sensor surfaces 20 as a function of an adjustable height of the spacer 14. The spacer 14 includes a means for changing a proximity of the sensor surface 20 to the skin surface 22 via the adjustable height (or thickness) 24. During night-time sleep measurements, the adjustable height 24 is changed from a first height, indicated by T1 in Fig. 1, to a second height, indicated by T2 in Fig. 1, greater than the first height. At the second height, a contact pressure between the sensor surface 20 and the skin surface 22 is lower and/or separation distance is higher which results in a slightly higher amount of transmitted ambient light to the optical sensor 18. During other than night-time sleep measurements, the adjustable height 24 is changed to the first height T1, wherein the contact pressure between the sensor surface and the skin surface is higher and/or separation distance is lower which results in a slightly lower amount of transmitted ambient light to the optical sensor. In one embodiment, the first height T1 is 1 mm or smaller than 1 mm (such as 0.5 mm or 0.1 mm) and the second height T2 is greater than 1mm, up to 2 mm.

In one embodiment, the means for changing the proximity of the sensor surface 20 to the skin surface 22 via the adjustable height comprises an actuator 28 configured to adjust a thickness of the spacer 14 between a first thickness T1 and a second thickness T2, larger than the first thickness. The actuator 28 (i) expands the spacer thickness to the second thickness in low ambient lighting conditions and/or a detected sleep condition and (ii) retracts the spacer thickness to the first thickness T1, in other than low ambient lighting conditions and/or the detected sleep condition. In one embodiment, the actuator 28 is controlled, via the controller 16. as a function of a background optical signal of the optical sensor 18. In another embodiment, the actuator 28 comprises an optically active smart material which is responsive to an optical input (e.g., of ambient light level or intensity). Examples of optically active smart materials may include photostrictive materials such as Lead Lanthanum Zirconate Titanate (PLZT) ceramics and various organic polymers such as poly(ethylacrylate) compounds with azo-aromatic crosslinks, some types of liquid crystalline polymers and certain elastomers.

With reference still to Fig. 1, the controller 16 is configured for controlling at least one physiological or sleep related measurement modality of the sleep tracker wearable device 10. The physiological and/or sleep related measurement modality can include at least one modality that utilizes the optical sensor/emitter assembly 12 for emitting and receiving optical signals The controller 16 is coupled to the optical sensor/emitter assembly 12 (i) for detecting background lighting conditions based on the optical signals being representative of background lighting conditions including ambient light and (ii) for removing artefacts in the optical signals. The artefacts can be attributable to an external interaction with or perturbation of a natural progression of ambient lighting level from movement or repositioning of the sleep tracker wearable device during operation.

Also shown in Fig. 1 are a position sensor 26 and an actuator 28, as will be discussed further herein. The position sensor 26 is electrically coupled to the controller 16. The position sensor can be configured to provide information on an angular orientation of the sleep tracker wearable device. In this embodiment, the controller 16 is further configured for correcting the optical signals for ambient light levels based on the angular orientation information. The position sensor 26 may comprise one or more of accelerometer, a gyroscope, an inertial measurement unit (IMU), or any combination thereof. An IMU describes a collection of measurement tools. When installed in the wearable device 10, these tools can capture data about the device's movement. IMUs contain sensors such as accelerometers, gyroscopes, and magnetomerters. The accelerometer may comprise, for example, a 3-axis or 6-axis accelerometer.

In a further embodiment, the means for changing the proximity of the sensor surface to the skin surface via the adjustable height comprises actuator 28. The actuator 28 may be configured to adjust a thickness of the spacer between a first thickness T1 and a second thickness T2, larger than the first thickness. The actuator 28 (i) expands the spacer thickness to the second thickness in low ambient lighting conditions and/or a detected sleep condition and (ii) retracts the spacer thickness to the first thickness, in other than low ambient lighting conditions and/or the detected sleep condition. In one embodiment, the actuator 28 is controlled as a function of a background optical signal of the optical sensor 18. In another embodiment, the actuator 28 comprises an optically active smart material which is responsive to an optical input (e.g., of ambient light level or intensity).

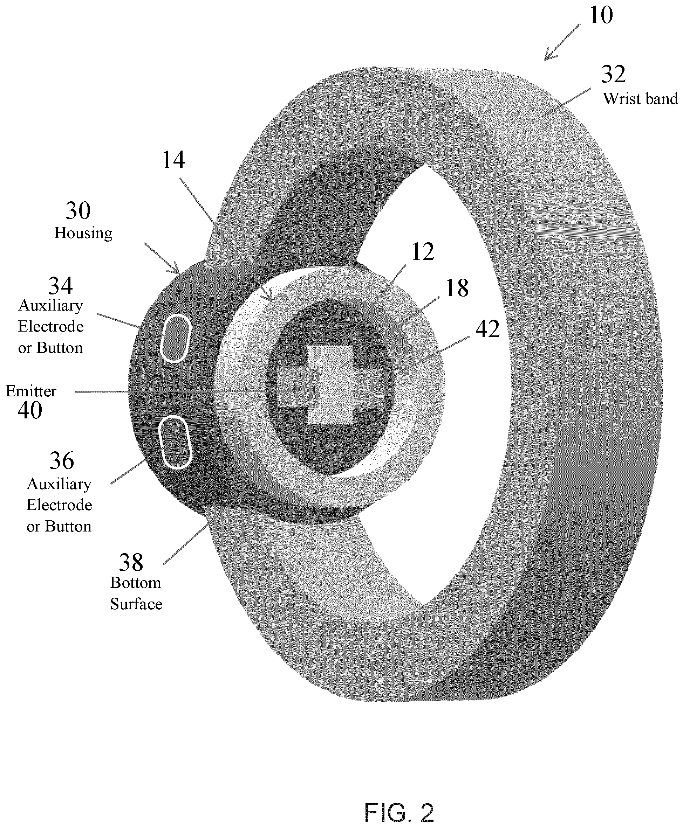

With reference now to Fig. 2, a schematic perspective view of the sleep tracker wearable device 10 with ambient light artefact detection and removal according to an embodiment of the present disclosure is shown. In addition to spacer 14, the wearable device 10 includes a housing 30, a wrist band 32 coupled to the housing 30, wherein the wrist band 32 is used to removably secure the wearable device 10 to the wrist (not shown) of a subject when being worn by the subject. Wrist band 32 could include any suitable material and latch mechanism (not shown) for adjusting how tight the wearable device is held to the subject's wrist. In another embodiment, a wearable band or patch (not shown) is coupled to the housing 30 for securing the housing to the skin of the subject while the wearable device 10 is being worn by the subject. In yet another embodiment, the wearable band comprises one selected from the group consisting of a head band, wrist band, an ankle band, a chest band, and a waist band.

Wearable device 10 may further include auxiliary electrodes, knobs and/or buttons 34 and 36 positioned on an outer side surface of housing 30. In one embodiment, the auxiliary electrodes are coupled to a side surface of the housing, other than the bottom surface 38, wherein the at least one of the electrodes is electrically coupled to the controller 16 for obtaining electrical measurements according to the at least one measurement modality during operation of the wearable device. In one embodiment, the auxiliary electrodes 34 and 36 are disposed on a same side surface of the housing 30.

In the illustration of Fig. 2, according to one embodiment, the spacer 14 has an outer perimeter surface that is at a diameter which is less than a diameter of a corresponding outer perimeter surface of the housing 30 upon which the spacer is mounted. In another embodiment, the outer perimeter surface of the spacer 14 can be aligned with the outer perimeter surface of the housing 30. In addition, the spacer 14 has a height dimension which is configured for spacing the sensor/emitter assembly 12 at a given contact pressure and/or separation distance with respect to the skin surface of subject during use of wearable device 10 while worn by the subject. The sensor/emitter assembly 12 further includes one or more emitters 40 and 42.

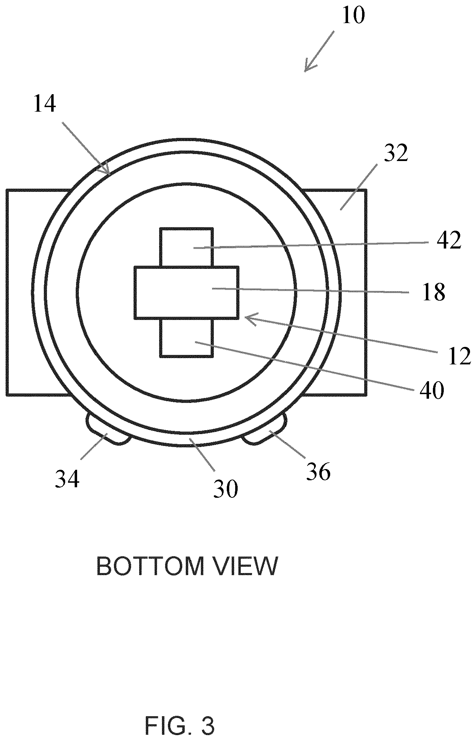

With reference now to Fig. 3, there is shown a bottom view of the sleep tracker wearable device 10 with ambient light artefact detection and removal according to an embodiment of the present disclosure. The sensor/emitter assembly 12 includes a sensor 18 and emitters 40, 42 disposed on opposite sides of the sensor 18. In one embodiment, the sensor/emitter assembly 12 is configured for obtaining an optical measurement (e.g., SpOz) that requires the spacer 14 to shield the optical sensor 18 from ambient light interference in order to derive a reliable PPG-based measurement, such as heart rate and oxygen saturation SpO₂. For example, SpO₂, also known as oxygen saturation, is a measure of the amount of oxygen-carrying hemoglobin in the blood relative to the amount of hemoglobin not carrying oxygen. The body needs there to be a certain level of oxygen in the blood or it will not function as efficiently.

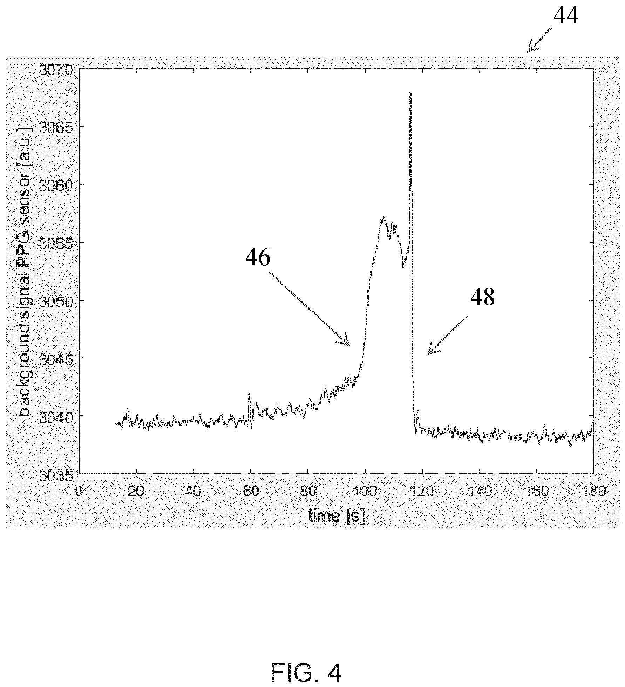

With reference now to Figs. 1 and 4, in one embodiment, the controller 16 may further be configured for distinguishing between (i) short-term optical signal changes due to a user movement and (ii) rapid external optical signal changes due to other than the user movement. In addition, the controller 16 is configured for improving a quality of ambient light meta-data derived from the optical sensor signal based on the distinction between the short-term optical signal changes and the rapid external optical signal changes. If long-term (slow rate) changes are detected, the user may have poor or inadequate daylight shutters and can be prompted with advice (e.g., via an AI algorithm as discussed further herein) on how to improve his or her bedroom environment to achieve a desired light level for improved sleeping. It is noted that in this embodiment absolute light levels are not detected (i.e., the embodiment is not configured to detect absolute light levels).

Fig. 4 is a graph view illustration 44 of an output signal of the optical sensor illustrating a difference between a long-term change in the optical signal and a short abrupt change in the optical signal according to an embodiment of the present disclosure. In particular, Fig. 4 is representative of a sensor output from the optical unit of the sleep tracker wearable device 10 (e.g., implemented in the form of a PPG watch) under daylight conditions. The vertical axis is representative of background optical signal of a PPG sensor in arbitrary units (a.u.) versus time on the horizontal axis in units of seconds (s). A long-term change 46 is noted from the time of 60-115 seconds, wherein a gradual change in the background optical signal is measured due to sun coming out from behind the clouds. A short abrupt change 48 is noted at about 118 seconds, wherein the user moves his or her wrist to a shady area from a brighter area (i.e., abrupt change).

For example, the method of detecting long-term change and short abrupt change can be implemented by applying a low pass filter in combination with an event detector over the background signal of the optical sensor 18 (e.g., a PPG sensor). The event detector is a based on a high pass filter in combination with a threshold value to detect movement spikes. The threshold value can be calibrated or determined as a global setting (e.g., related to the floor noise level of the sensor 18).

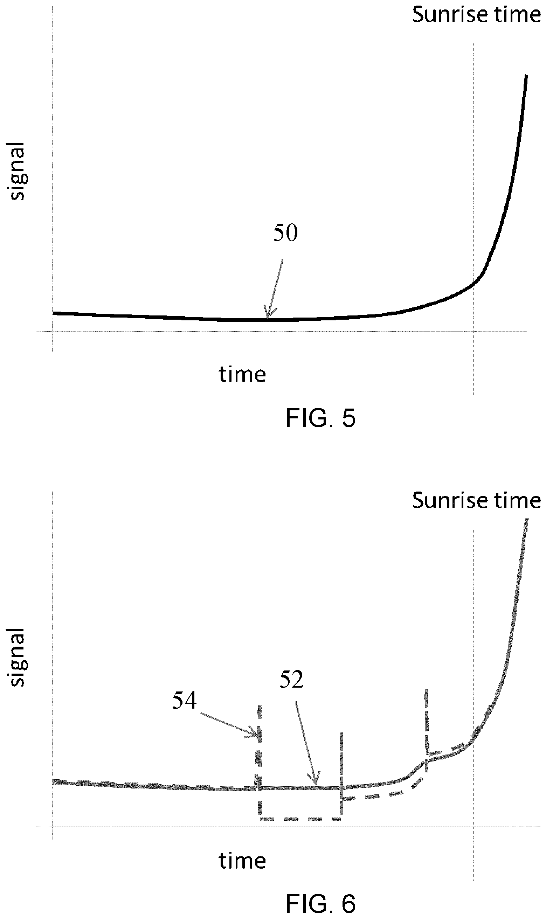

There are at least two possible cases of signal change that include: (1) in the absence of movement, and (2) in the presence of movement. In absence of movement, no spikes are detected and the background light level will gradually increase with time as the ambient light levels increase. If the increase corresponds with daylight data (i.e., sunrise timing) the system recognizes this as influence from outside light conditions. In presence of movement: spikes are detected after which the ambient light levels are adjusted for continuity (i.e., match the levels from before and after the movement, within a given time interval), so that the data remains substantially continuous and can be compared to the daylight data as in the first case (1). Fig. 5 is a graph view illustration of an output signal of the optical sensor 18 as a function of time for a case without movement of the sleep tracker wearable device 10 according to an embodiment of the present disclosure. In particular, Fig. 5 is representative of the sensor output signal 50 as a function of time for case (1) without movement. Fig. 6 is a graph view illustration of an output signal of the optical sensor 18 as a function of time for a case of movement of the sleep tracker wearable device 10, and a stitched signal, according to an embodiment of the present disclosure. In particular, the dotted line in Fig. 6 is representative of the sensor output signal 54 (with varying levels and movement spikes) for case (2) with some movement. The solid line 52 in Fig. 6 represents the processed (stitched) signal.

In another embodiment of the present disclosure, the device and method comprise a wearable device 10 with artefact detection using an optical sensor 18 with improved signal quality. In connection with the first embodiment, the example given has a relatively large change in light level (Fig. 4). Smaller changes in light level may fall under the noise floor of the sensor 18, depending on how tight the wearable device 10 is worn. This also poses a significant influence of the exact position of the sensor 18 with respect to the user's skin, whereby a small difference in contact (pressure and/or separation distance) may influence the amount of ambient light that reaches the sensor, which makes it difficult to calibrate the system.

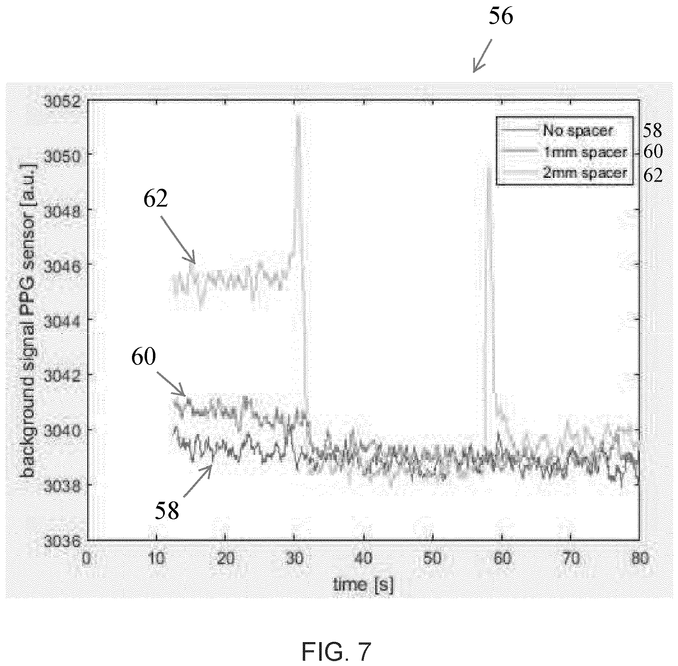

To provide improved signal quality, the device and method according to another embodiment reduces the proximity of the sensor 18 to the skin 22 during nighttime measurements by the spacer 14 between the skin and the sensor surface. In daytime and during activity, this is an unwanted situation due to the higher amount of ambient light entering the optical sensor 18 (which reduces signal to noise ratio), however during nighttime darkness, this is much less of a problem. The effect of adding the spacer 14 is shown in Fig. 7. The optical unit or sensor/emitter assembly 12 is still contacting the skin 22 when a spacer 14 up to 2mm is added. However, the contact pressure is lower which results in a slightly higher amount of transmitted ambient light. From the signal with 1mm or 2mm spacer it is possible to distinguish low lighting conditions (overcast daytime sky) with darkness (covered by heavy blanket).

With reference still to Fig. 7, a graph view illustration of sensor output from an optical sensor of a sleep tracker wearable device 10 under daylight conditions (overcast sky). In particular, Fig. 7 is representative of a sensor output from the optical unit 12 of the sleep tracker wearable device 10 (e.g., implemented in the form of a PPG watch) under daylight conditions. The vertical axis is representative of background optical signal of a PPG sensor in arbitrary units (a.u.) versus time on the horizontal axis in units of seconds (s). In Fig. 7, from 10-30 seconds the wearable device 10 is on the user's wrist facing a window, from 30-60 seconds the wearable device is covered by placing the wearable device under a heavy blanket, and after 60 seconds the blanket is removed and the wearable device 10 is held in an opposite direction (i.e., facing away from the window, according to an embodiment of the present disclosure. The three signal curves shown in Fig. 7 are representative of the wearable device 10 with no spacer (curve 58), a 1 mm spacer (curve 60), and a 2 mm spacer (curve 62).

The spacer 14 can be activated during nighttime only to avoid detrimental effects on the signal quality during daytime and activity. For instance, the spacer 14 can be activated by a controllable switch so the user can manually activate the spacer before going to sleep (for instance by a button press that releases a spring-loaded spacer geometry or via turning a knob which makes the spacer geometry protrude from the wearable surface). Alternatively, the spacer activation can be coupled to a manual sleep intent marker that may be present on the wearable device 10.

The embodiment with the adjustable spacing of the spacer 14 is more suitable for determining overall lighting conditions during nighttime (i.e., after sunset) so can also provide input for feedback on general nighttime lighting conditions (e.g., does the user leave a light on at night which is too bright?).

In another embodiment, the spacer height is automatically made dynamic, for instance by employing an actuator 28 which makes the spacer 14 thicker or thinner when needed but without compromising the primary sensor function. For instance, in low ambient lighting conditions and/or detected sleep, the spacer will expand from a first thickness setting to a second, thicker thickness setting, allowing a higher background signal (so it is easier to distinguish differences). After waking when the sun is up/light is on, the spacer becomes thinner (returns to the first thickness setting) to guarantee a high-quality optical heart rate (HR) signal measurement. The spacer 14 could be actuated by a small actuator 28 that is controlled, via controller 16, as function of the background light signal of the optical sensor 18 (e.g., a PPG sensor) or made of smart material that responds to an optical input (i.e., optically active materials).

The device and method according to yet another embodiment can comprise a wearable device with relative ambient lighting monitoring with artefact removal using an optical sensor and accelerometer. In other words, the ambient light level measurement can be augmented by including position sensing via an accelerometer. A 3-axis or 6-axis accelerometer can provide information on the (angular) orientation of the wearable device 10, which can be used to correct the signal measured by the wearable device for the ambient light levels. For instance, as can be seen in Fig. 7, there is a difference in signal due to orientation towards the light source.

Also coupling the signal of the accelerometer can help distinguish short-term signal changes from user movement and rapid external changes (e.g., turning on lights or opening curtains by another person), which can improve the quality of the ambient light meta-data derived from the sensor signal.

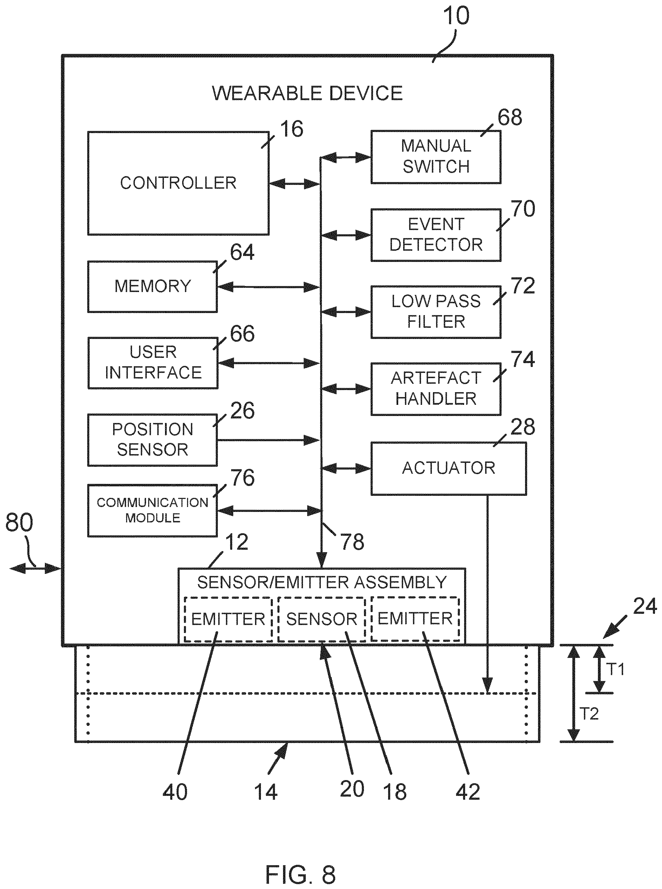

With reference now to Fig. 8, there is shown a block diagram view of the wearable device 10 with ambient light artefact detection and removal according to another embodiment of the present disclosure. The wearable device 10 is similar to the embodiment shown in Fig. 1 with the following differences. The wearable device 10 can further include one or more of a memory 64, user interface 66, manual switch 68, event detector module 70, low pass filter module 72, artefact handler module 74 and a communications module 76 (or communication means).

The user interface 66 may comprise any suitable user interface operatively coupled to at least the controller 16, via signal lines 78, for use in connection with the embodiments as discussed herein. For example, user interface 66 can comprise at least one selected from the group consisting of an input/output device, a tactile output device, a touch screen, an optical display, a microphone, a keypad, a keyboard, a pointing device, an image capture device, a video camera, an audio output device, and any combination thereof, determined as appropriate according to the requirements of a given sleep tracker wearable device implementation and/or application.

The communications module 76 or communication means is configured for wired or wirelessly communicating, bi-directionally, with at least one communication means (not shown) located remote from the wearable device 10. The communications module 76 is further for receiving commands and/or data appropriate for a given sleep tracker wearable device implementation. Communications module 76 is preferably a low-power short-range transceiver or wireless or wired connection. In one embodiment, the communication means or module 76 comprises a communication device configured to communicate via one or more of a passive RFID tag, SPI, Dual SPI, Quad SPI, UART, I2C, Single Wire/1-wire, HSL, Parallel Flash, USB, NFC, RFID, Bluetooth, Fiber optic, Zigbee/ZWAVE, IRDA, Wi-Fi, or other similar type of communication protocol.

Communication between the communication module 76 of the wearable device 10 and a remote device is indicated by reference numeral 80. In other words, communication between the various devices and components as discussed herein is preferably accomplished using suitable near-field communication techniques known in the art, and thus are not discussed further herein.

The controller 16 operatively couples to the user interface 66 and the communication module 76 via suitable signal lines, indicated via reference numeral 78. Controller 16 is configured for operating in response to at least one of a power up sequence, via ON/OFF switch 68, and/or an activation sequence, via the user interface 66, to perform according to a given wearable device sleep tracking implementation. as discussed herein.

In one embodiment, controller 16 comprises one or more of a microprocessor, microcontroller, field programmable gate array (FPGA), integrated circuit, discrete analog or digital circuit components, hardware, software, firmware, or any combination thereof, for performing various functions as discussed herein, further according to the requirements of a given sleep tracker wearable device implementation and/or application. Controller 16 can further comprise one or more of various modules, units, or subsystems as discussed herein.

With reference still to Fig. 8, each of the one or more of the memory 64, user interface 66, manual switch 68, event detector module 70, low pass filter module 72, artefact handler module 74 and a communications module 76 is operatively coupled to at least the controller 16, e.g., via signal lines 78. The switch 68 comprises any suitable switch for powering the wearable device 10 between ON and OFF. The event detector module 70 comprises any suitable computer program module or circuit for detecting optical signal events as discussed herein for a given sleep tracker wearable device implementation and/or application. The low pass filter module 72 comprises any suitable computer program module or circuit for low pass filtering of optical signals as discussed herein for a given sleep tracker wearable device implementation and/or application. The artefact handler module 74 comprises any suitable computer program module or circuit for handling artefacts as discussed herein for a given sleep tracker wearable device implementation and/or application. It is understood that the described modules may be computer program modules which are rendered in a non-transitory computer-readable medium. In addition, the modules discussed herein may comprise one or more of an integrated circuit, discrete analog or digital circuit components, hardware, software, firmware, or any combination thereof, for performing various functions as discussed herein, further according to the requirements of a given sleep tracker wearable device implementation and/or application. In addition, one or more of the modules 70, 72 and 74 can further comprise various combinations of one or more of the various modules. It is understood that the described modules may be computer program modules which are rendered in a non-transitory computer-readable medium.

In one embodiment, memory 64 can comprise any suitable memory device, operatively coupled to at least the controller 16, for at least storing information thereto, and further for at least subsequently retrieving the information there from. Memory 64 is preferably a somewhat persistent and very low-power volatile memory, such as flash memory, to which data can be automatically written, stored, and subsequently retrieved for use in a given sleep tracker wearable device implementation and/or application. According to a still further embodiment, the sleep tracker wearable device 10 further comprises a visual indicator and an audio output, e.g., via user interface 66, in communication with the computer hardware controller 16.

According to one embodiment, the controller 16 is further configured for implementing an artificial intelligence algorithm for outputting targeted user-specific sleep advice (e.g., of a highest value) based on (i) sleep data obtained via the at least one measurement modality, (ii) personal data coupled to the sleep data, and (iii) meta-data relating to surroundings of the sleep tracker wearable device based on the detected background lighting conditions.

The embodiments of the present disclosure provide various advantages. For example, there are many more users who wear a sleep tracker than people with a dedicated bedside light sensor, therefore if sleep tracker wearables could provide an input on the ambient lighting conditions this could provide valuable metadata on the surroundings. As discussed herein, the sleep tracker wearable 10 comprises an optical sensor configured to sense ambient lighting conditions, and a controller, responsive to the sensed ambient lighting conditions, configured to generate metadata relating to surroundings of the wearable device. This metadata will become more and more crucial as input for AI assisted algorithms that provide feedback and advice on sleep in consumer settings as well as medical settings.

The system and method according to the embodiments of the present disclosure advantageously provide a simple way of using the existing sensors' background signal to obtain information on changes in ambient lighting levels in environment surroundings. The obtained information can be used advantageously to provide meaningful feedback to users of consumer sleep wearables or to caregivers interpreting medical wearables data.

As an example, the AI algorithm may use the ambient light measurements over an extended period of time (e.g., several weeks) as an input, next to the sleep quality. For example, one measure could be if the ambient lighting conditions increase towards the end of some nights which results in a decrease in sleep quality (i.e., more fragmentation in sleep stages towards the end of the sleep period), as compared to other nights where the ambient light levels do not increase towards the morning. If the increase in lighting levels correlates with sleep fragmentation (and optionally additional information from publicly known sunrise data, user location data and subjective sleep quality from user), then the AI algorithm might advise to improve light blocking quality of the curtains or shutters to improve sleep quality. In another embodiment, substitute sunrise for sunset and look at sleep quality at the beginning of the night.

Another example of AI algorithm is to compare the ambient light data with publicly available sunrise data to determine whether the environmental lighting conditions are natural (sunrise correlated) or artificial (i.e. user is using a (non-connected) Philips wake up light or other (non-connected) wake up lights that mimic sunrise lighting and give advice on the light settings for such devices depending on the detected sleep quality of the user (for instance lowering the settings if the user is highly sensitive or increasing or decreasing the wake up lights intensity ramping speed based on sleep quality in the time prior to waking).

Although only a few exemplary embodiments have been described in detail above, those skilled in the art will readily appreciate that many modifications are possible in the exemplary embodiments without materially departing from the novel teachings and advantages of the embodiments of the present disclosure. For example, the embodiments of the present disclosure can be advantageously used in any application or system which combines sleep tracking with meta-data from the night or that may feed into an application using algorithms that are refined by AI using a combination of measured sleep parameters and ambient surrounding parameters. Accordingly, all such modifications are intended to be included within the scope of the embodiments of the present disclosure as defined in the following claims. In the claims, means-plus-function clauses are intended to cover the structures described herein as performing the recited function and not only structural equivalents, but also equivalent structures.

In addition, any reference signs placed in parentheses in one or more claims shall not be construed as limiting the claims. The word "comprising" and "comprises," and the like, does not exclude the presence of elements or steps other than those listed in any claim or the specification as a whole. The singular reference of an element does not exclude the plural references of such elements and vice-versa. One or more of the embodiments may be implemented by means of hardware comprising several distinct elements, and/or by means of a suitably programmed computer. In a device claim enumerating several means, several of these means may be embodied by one and the same item of hardware. The mere fact that certain measures are recited in mutually different dependent claims does not indicate that a combination of these measures cannot be used to an advantage.

## Claims

1. A sleep tracker wearable device (10) with ambient light artefact detection and removal when worn by a user, comprising:
an optical sensor/emitter assembly (12) having an optical sensor (18) with a sensor surface (20) configured for receiving optical signals including ambient light;
a spacer (14) configured for being disposed between the sensor surface of the optical sensor/emitter assembly and a skin surface (22) of the user during operation, wherein the spacer is configured for providing a given contact pressure, separation distance, or a combination of contact pressure and separation distance between the skin and sensor surfaces as a function of an adjustable height (24) of the spacer; and
a controller (16) configured for controlling at least one measurement modality of the sleep tracker wearable device, wherein the controller is coupled to the optical sensor/emitter assembly (i) for detecting background lighting conditions based on the optical signals and (ii) for removing artefacts in the optical signals.

2. The sleep tracker wearable device (10) according to claim 1, wherein further the controller (16) is configured for applying a low pass filter (72) in combination an event detector (70) over a background optical signal of the optical sensor, wherein the event detector is a based on a high pass filter in combination with a threshold value to detect movement spikes in the background optical signal.

3. The sleep tracker wearable device (10) according to claim 2, wherein the threshold value is calibrated or determined as a global setting related to a floor noise level of the optical sensor (18).

4. The sleep tracker wearable device (10) according to claim 2, wherein in an absence of movement, no spikes in monitored ambient light levels are detected and a background light level will gradually increase with time as the ambient light levels increase, further wherein the controller (16) determines if the increase in ambient light level corresponds with daylight data as influenced from outside light conditions in contrast to ambient light conditions other than daylight.

5. The sleep tracker wearable device (10) according to claim 2, wherein in a presence of movement, spikes in monitored ambient light levels are detected after which the controller adjusts the detected ambient light levels for continuity, so that the ambient light level data remains continuous and can be compared to the daylight data, wherein the controller (16) determines if the increase in ambient light level corresponds with daylight data as influenced from outside light conditions in contrast to ambient light conditions other than daylight.

6. The sleep tracker wearable device (10) according to claim 1, wherein the spacer (14) includes a means (28) for changing a proximity of the sensor surface (20) to the skin surface (22) via the adjustable height (24), wherein (i) during night-time sleep measurements, the adjustable height is changed from a first height (T1) to a second height (T2) greater than the first height, wherein a contact pressure between the sensor surface (20) and the skin surface (22) is lower and/or separation distance is higher which results in a slightly higher amount of transmitted ambient light to the optical sensor (18) and (ii) during other than night-time sleep measurements, the adjustable height (24) is changed to the first height (T1), wherein the contact pressure between the sensor surface and the skin surface is higher and/or separation distance is lower which results in a slightly lower amount of transmitted ambient light to the optical sensor.

7. The sleep tracker wearable device (10) according to claim 6, wherein the spacer (14) comprises one selected from the group consisting of (i) a spring-loaded spacer geometry, (ii) a manually activated spacer geometry, and (iii) a spacer geometry coupled to a manual sleep intent marker.

8. The sleep tracker wearable device (10) according to claim 6, wherein the means for changing the proximity (28) comprises an actuator configured to adjust a thickness of the spacer between a first thickness (T1) and a second thickness (T2), larger than the first thickness, wherein the actuator (i) expands the spacer thickness to the second thickness in low ambient lighting conditions and/or a detected sleep condition and (ii) retracts the spacer thickness to the first thickness, in other than low ambient lighting conditions and/or the detected sleep condition.

9. The sleep tracker wearable device (10) according to claim 1, further comprising:
a position sensor (26) configured to provide information on an angular orientation of the sleep tracker wearable device, wherein the controller (16) is further configured for correcting the optical signals for ambient light levels based on the angular orientation information.

10. The sleep tracker wearable device (10) according to claim 9, wherein the controller (16) is further configured for distinguishing between (i) short-term optical signal changes due to a user movement and (ii) rapid external optical signal changes due to other than the user movement, and for improving a quality of ambient light meta-data derived from the optical sensor signal based on the distinction between the short-term optical signal changes and the rapid external optical signal changes.

11. The sleep tracker wearable device (10) according to claim 1, wherein the controller (16) is further configured for implementing an artificial intelligence algorithm for outputting targeted user-specific sleep advice based on (i) sleep data obtained via the at least one measurement modality, (ii) personal data coupled to the sleep data, and (iii) meta-data relating to surroundings of the sleep tracker wearable device based on the detected background lighting conditions.

12. A method for ambient light artefact detection and removal in a sleep tracker wearable device, comprising:
receiving, via an optical sensor/emitter assembly having an optical sensor with a sensor surface, optical signals including ambient light;
disposing a spacer between the sensor surface of the optical sensor/emitter assembly and a skin surface of the user during use, wherein the spacer is configured for providing a given contact pressure and/or separation distance between the skin and sensor surfaces as a function of an adjustable height of the spacer; and
controlling, via a controller coupled to the optical sensor/emitter assembly, at least one measurement modality of the sleep tracker wearable device, wherein controlling includes (i) detecting background lighting conditions based on the optical signals and (ii) removing artefacts in the optical signals attributable to an external interaction with or perturbation of a natural progression of ambient lighting resulting from movement or repositioning of the sleep tracker wearable device during operation.

13. The method according to claim 12, wherein disposing the spacer includes changing a proximity of the sensor surface to the skin surface via the adjustable height, wherein (i) during night-time sleep measurements, the adjustable height is changed from a first height to a second height greater than the first height, wherein a contact pressure between the sensor surface and the skin surface is lower and/or separation distance is higher which results in a slightly higher amount of transmitted ambient light to the optical sensor and (ii) during other than night-time sleep measurements, the adjustable height is changed to the first height, wherein the contact pressure between the sensor surface and the skin surface is higher and/or separation distance is lower which results in a slightly lower amount of transmitted ambient light to the optical sensor.

14. The method according to claim 12, further comprising:
configuring a position sensor to provide information on an angular orientation of the sleep tracker wearable device; and
correcting, via the controller, the optical signals for ambient light levels based on the angular orientation information.

15. The method according to claim 12, further comprising:
implementing, via the controller, an artificial intelligence algorithm for outputting targeted user-specific sleep advice based on (i) sleep data obtained via the at least one measurement modality, (ii) personal data coupled to the sleep data, and (iii) meta-data relating to surroundings of the sleep tracker wearable device based on the detected background lighting conditions.
